# FASCICULE DE BREVET EUROPEEN

(11) **EP 3 413 787 B1**
(45) Date de publication et mention de la délivrance du brevet: **11.05.2022**
(21) Numéro de dépôt: 17708172.6
(22) Date de dépôt: 13.02.2017
(51) Int. Cl.: A61B 5/00, A61N 1/36

(54) **SYSTÈME COMPRENANT UNE SONDE POUR DÉTECTER UNE DÉCHARGE MASSIVE DE POTENTIELS D'ACTION ET UNE SONDE POUR STIMULER UN NERF VAGUE D'UN PATIENT ÉPILEPTIQUE**
SYSTEM UMFASSEND EINE SONDE ZUR DETEKTION EINER MASSIVEN ENTLADUNG VON AKTIONSPOTENZIALEN UND EINE SONDE ZUR STIMULATION EINES VAGUSNERVS EINES EPILEPTISCHEN PATIENTEN
SYSTEM COMPRISING A LEAD FOR DETECTING A MASSIVE ACTION POTENTIAL DECHARGE AND A LEAD FOR STIMULATING A VAGUS NERVE OF AN EPILEPTIC PATIENT

(30) Priorité: 12.02.2016 FR 1670039
(43) Date de publication de la demande: 19.12.2018
(73) Titulaire: Université de Rennes, 35000 Rennes (FR); Institut National de la Santé et de la Recherche Médicale (INSERM), 75013 Paris (FR); CNRS Centre National de la Recherche Scientifique, 75016 Paris (FR); CHU De Rennes (Centre Hospitalier Universitaire De Rennes), 35000 Rennes (FR)
(72) Inventeur: HERNANDEZ, Alfredo, 35510 Cesson-sevigne (FR); MARTIN, Benoit, 45130 Meung-sur-loire (FR); BIRABEN, Arnaud, 35700 Rennes (FR)
(74) Mandataire: Le Forestier, Eric
(86) Numéro de dépôt international: PCT/EP2017/053156
(87) Numéro de publication internationale: WO 2017/137627

(56) Documents cités:
- US-A1- 2006 052 831
- US-A1- 2007 027 486
- US-A1- 2007 255 320
- US-A1- 2014 316 497
- US-B1- 6 658 287

## Description

### Domaine de l'invention

La présente invention concerne le domaine des dispositifs médicaux destinés à prévenir les conséquences les plus graves d'une crise d'épilepsie chez l'humain.

Plus particulièrement elle concerne un dispositif médical visant, en s'appuyant sur l'activité d'au moins un nerf vague et de préférence des deux nerfs vagues, à identifier la survenance d'une activité anormale pouvant être létale, secondaire à une crise d'épilepsie (phénomène SUDEP pour « Sudden Unexpected Death in EPilepsy » en terminologie anglo-saxonne), et à diminuer ce risque en agissant sur le(s) nerf(s) vague(s) pour enrayer le phénomène.

### Etat de la technique

Aujourd'hui, on ne sait pas prévenir le risque de mort subite, ni enrayer le phénomène d'épilepsie susceptible de causer la mort subite lorsqu'il survient.

L'état de l'art le plus proche est représenté par les documents suivants.
a) Le document US2014/0142653A1 décrit un procédé pour traiter un patient souffrant d'épilepsie, basé sur la mesure d'un signal physiologique (typiquement un électroneurogramme, éventuellement en combinaison avec la mesure de la fréquence cardiaque) permettant de déterminer si le patient est en train de subir une crise, ou vient de subir une crise. En fonction de la mesure obtenue, le procédé applique une stimulation sur l'un des deux nerfs vagues, ou sur les deux, d'une part dans le sens efférent pour agir sur la fréquence cardiaque, et d'autre part dans le sens afférent pour tenter de réduire le phénomène épileptique. Ce document ne donne toutefois aucune indication sur la façon dont un risque effectif de mort subite causé par l'épilepsie peut être déterminé, ni sur la façon dont une stimulation pourrait réduire voire annuler ce risque, ni même sur la capacité du procédé à réduire la mortalité autrement qu'en réduisant les crises d'épilepsie. Ainsi il concerne uniquement le traitement des crises d'épilepsie de façon générale.
b) Le document US8634922B1 enseigne un système de stimulation du nerf vague pour le traitement de l'épilepsie, qui prend en compte une mesure du signal cardiaque pour optimiser le traitement, et plus précisément pour corriger une stimulation destinée à traiter une crise d'épilepsie afin de prévenir des effets indésirables au niveau de la fréquence cardiaque. Par ailleurs, selon ce document, seule une stimulation dans le sens afférent est appropriée pour traiter la crise d'épilepsie.

Il existe par ailleurs de nombreux autres documents sur la stimulation des nerfs vagues, mais aucun d'eux ne traite spécifiquement de la prévention du risque de mort subite lors de la survenance d'une crise d'épilepsie. On citera notamment US 2007/027486 A1, qui mentionne une stimulation des nerfs vagues dans le sens afférent, et US 2007/255320 A1. On citera également US 6 658 287 B1 qui vise à prédire la survenance d'une crise d'épilepsie principalement par une analyse sophistiquée de signaux d'électro-encéphalogram me.

Il existe enfin des solutions commerciales de stimulation du nerf vague qui visent à diminuer le nombre de crises d'épilepsie chez les patients épileptiques, mais ces solutions sont inefficaces pour protéger les patients contre la mort subite si une crise survient. Un exemple d'un tel système de neurostimulation du nerf vague est commercialisé par la société Cyberonics et est indiqué pour l'épilepsie invalidante ou pharmaco-résistante.

### Résumé de l'invention

La présente invention vise à permettre d'une part la détection au niveau d'un nerf vague ou de préférence au niveau des deux nerfs vagues, le cas échéant en combinaison avec un ou plusieurs autres signaux physiologiques, de la survenance d'une activité paroxystique anormale et intense, susceptible d'engendrer la mort (cette activité étant liée probablement à une crise d'épilepsie, mais l'objet de l'invention n'étant pas en soi de détecter la survenance de crises d'épilepsie), et d'autre part, en agissant au niveau d'un nerf vague ou de préférence au niveau des deux nerfs vagues, à réduire sensiblement ce risque d'hyperactivité et de décès.

On propose ainsi un système tel que défini dans la revendication 1.

Ce système comprend avantageusement mais facultativement les caractéristiques additionnelles des revendications dépendantes 2 à 15, prises individuellement ou en toutes combinaisons que l'homme du métier appréhendera comme étant techniquement compatibles.

### Brève description des dessins

D'autres aspects, buts et avantages de la présente invention apparaîtront mieux à la lecture de la description détaillée suivante d'une forme de réalisation préférée de celle-ci, donnée à titre d'exemple non limitatif et en référence aux dessins annexés, sur lesquels :
- la Figure 1 est une représentation schématique d'un système selon l'invention,
- la Figure 2 illustre un processus mis en oeuvre par le système selon l'invention,
- la Figure 3 donne un exemple de signaux recueillis sur un nerf vague et révélant un phénomène de décharge massive, et
- la Figure 4 donne un exemple de l'allure de signaux de stimulation appliqués à une sonde de stimulation d'un nerf vague.

### Description détaillée d'une forme de réalisation préférée

### a) Principe général

Le système selon l'invention a pour objet de prévenir les risques de décès liés aux crises d'épilepsie (essentiellement par le phénomène de mort subite « SUDEP »), d'une part grâce à une certaine caractérisation d'un signal électrophysiologique recueilli au niveau du nerf vague, de préférence au niveau des deux nerfs vagues, et d'autre part grâce à une certaine stimulation électrique, dans le sens efférent, appliquée au(x) nerf(s) vague(s).

La caractérisation est effectuée par détection d'une décharge massive dans le sens efférent du nerf vague, comme on va la décrire dans la suite, à l'aide d'une sonde placée autour de l'un des deux nerfs vagues ou de préférence de deux sondes placées autour des nerfs vagues gauche et droit.

Eventuellement, cette caractérisation peut être combinée à un ou plusieurs autres signaux physiologiques du patient, et notamment un signal cardiaque et/ou un signal respiratoire et/ou un signal d'oxymétrie et/ou un signal électromyographique.

L'action de diminution du risque de mort subite est effectuée selon l'invention par stimulation électrique sur l'un des nerfs vagues ou de façon préférée sur les deux nerfs vagues, dans des conditions propres à neutraliser les signaux véhiculés dans les nerfs. Cette neutralisation peut s'effectuer soit en appliquant au(x) nerf(s) une dépolarisation, de manière à provoquer une inhibition instantanée du fait de la période réfractaire, soit en appliquant au(x) nerf(s) une hyperpolarisation, ayant un effet de blocage dû à l'inhibition de l'excitabilité, pendant une durée contrôlée.

Pour déterminer les conditions optimales d'une dépolarisation ou d'une hyperpolarisation, on se référera de façon particulièrement utile au document « Conduction block in myelinated axons induced by high-frequency (kHz) non-symmetric biphasic stimulation », Z. Zhao et al., Frontiers in Computational Neuroscience, July 2015, Volume 9, Article 86, qui suggère la possibilité de recourir à des formes d'ondes électriques alternatives dissymétriques, avec une prépondérance de leur composante positive ou de leur composante négative, respectivement, pour aboutir à un blocage de la conduction des potentiels d'action dans les différentes fibres nerveuses.

Plus particulièrement, on peut recourir à une action électrique bi-phasique asymétrique, avec dans une première approche un signal alternatif ayant des impulsions positives plus longues que les impulsions négatives, pour créer une hyperpolarisation, et dans une deuxième approche un signal alternatif ayant des impulsions positives plus courtes que les impulsions négatives, pour créer une dépolarisation.

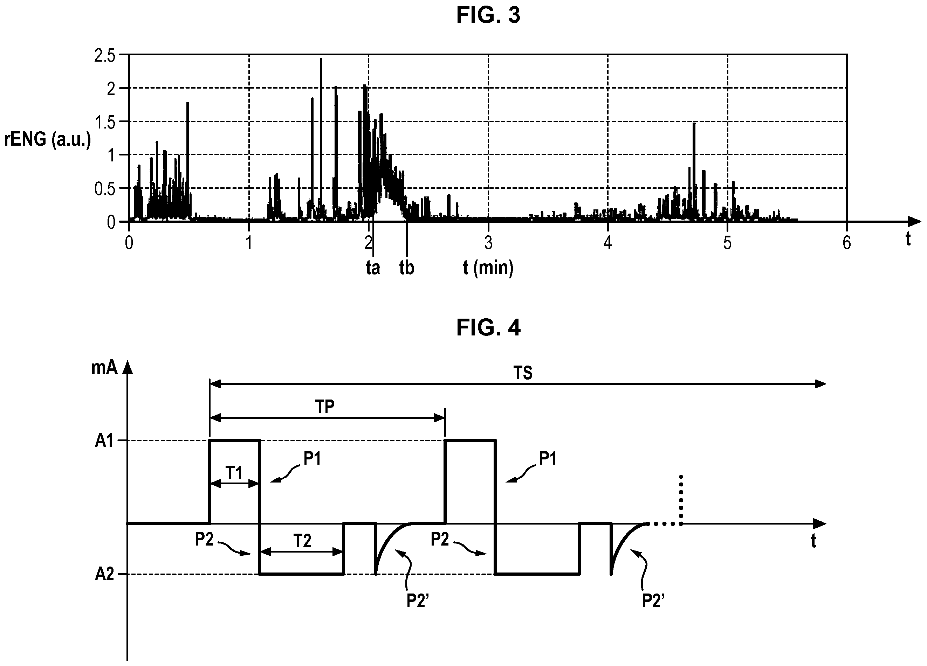

On donnera dans la suite en référence à la Figure 4 un exemple concret de signaux destiner à provoquer une hyperpolarisation du nerf vague.

Par ailleurs, il est possible de combiner de telles caractéristiques de signal électrique de stimulation avec le recours à des sondes à sélectivité spatiale. En particulier, on sait aujourd'hui, grâce à des structures d'électrodes de sonde particulières, appliquer une stimulation de façon préférentielle sur certaines sections du nerf vague, pour aboutir à cette sélectivité spatiale. La dépolarisation ou hyperpolarisation peut ainsi être appliquée de façon privilégiée sur certaines fibres nerveuses, en perturbant moins d'autres fibres dont la conduction des potentiels d'action n'a pas nécessairement à être bloquée. Cette sélectivité spatiale permet également de réduire l'amplitude du courant nécessaire pour provoquer un blocage de conduction.

Il est également possible d'utiliser la dimension temporelle en effectuant des dépolarisations et/ou hyperpolarisations selon différents cadencements dans différentes régions du ou des nerfs vagues.

### b) Mise en œuvre détaillée

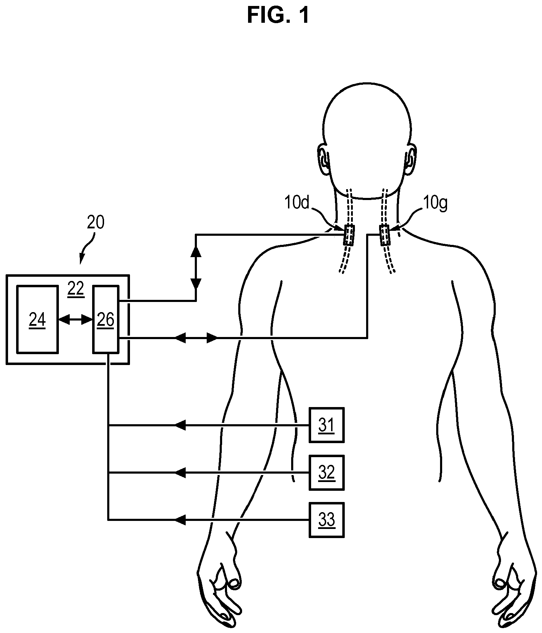

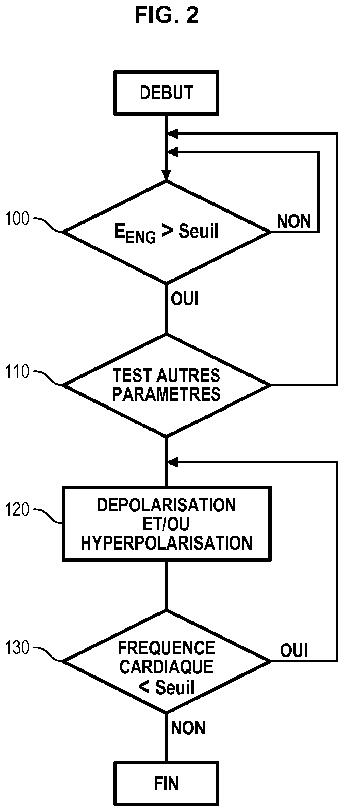

On va maintenant décrire en référence aux Figures 1 et 2 une mise en oeuvre plus détaillée de l'invention.

Sur la Figure 1, on a représenté les nerfs vagues gauche et droit d'un patient, respectivement NVG et NVD.

Sur chaque nerf vague a été implantée une sonde de détection et de neurostimulation, respectivement 10g et 10d. Chaque sonde comporte de préférence une matrice d'électrodes notamment pour réaliser une stimulation électrique avec sélectivité spatiale et/ou temporelle. Avantageusement, la combinaison d'électrodes, ou éventuellement une seule paire de cette combinaison, est utilisée également pendant les périodes d'absence de traitement pour effectuer la détection de décharge massive. Cette dernière peut être effectuée au niveau d'un seul nerf vague ou des deux nerfs vagues. Dans une forme de réalisation particulière, on peut mettre en oeuvre l'invention sur un seul des deux nerfs vagues. Notamment, le nerf vague droit étant essentiellement responsable des effets sur la fréquence cardiaque (noeud sinusal), on peut envisager une détection et une stimulation de dépolarisation et/ou d'hyperpolarisation seulement sur celui-ci. Toutefois la détection et la stimulation peuvent également être effectuées sur le nerf vague gauche, qui est notamment associé à la modulation du noeud auriculo-ventriculaire.

Les deux sondes sont reliées par des conducteurs à brins multiples à une unité 20, de préférence du type portable ou implantable, comportant dans un boîtier 22 une unité centrale de traitement 24 et des circuits d'entrée sortie 26 via lesquels les signaux sont appliqués aux sondes 10g, 10d et recueillis à partir desdites sondes. Une batterie non représentée alimente les circuits de l'unité 20.

Le système comprend également un capteur de fréquence cardiaque 31 tel qu'un capteur de pouls ou une sonde cardiaque, et le cas échéant un capteur respiratoire 32 et/ou un capteur d'oxymétrie 33.

Le capteur de fréquence cardiaque est utilisé au moins pour déterminer, comme on le verra dans la suite, si une neutralisation des potentiels d'action aboutit à maintenir le fréquence cardiaque au-dessus d'un seuil, c'est-à-dire si le risque d'arrêt cardiaque est écarté.

Il peut aussi être utilisé comme détecteur additionnel d'une crise d'épilepsie susceptible de conduire à une mort subite, en combinaison avec la détection d'une décharge massive dans au moins l'un des deux nerfs vagues. Cette détection additionnelle peut par exemple consister à déterminer si le fréquence cardiaque dépasse des seuils haut et bas prédéterminés, ces dépassements créant des situations à risque pour le patient. Elle peut également consister à détecter la présence des blocs auriculo-ventriculaires, qui peuvent être provoqués par une activation massive du nerf vague.

Le capteur respiratoire et/ou le capteur d'oxymétrie permet de déterminer si la fonction respiratoire du patient est remplie normalement. Dans le cas où un tel capteur est prévu, la détection d'une insuffisance respiratoire (diminution ou absence des mouvements musculaires de la respiration, ou baisse du taux d'oxygène dans le sang) est combinée à la détection d'une décharge massive à l'aide d'au moins l'une des sondes 10g, 10d pour identifier une crise d'épilepsie avec risque de mort subite.

Selon une autre amélioration, l'unité 20 peut comprendre des moyens pour analyser des signaux d'électromyographie de façon à détecter une activité musculaire anormale, due par exemple à la crise généralisée tonico-clonique souvent présente avant un événement de SUDEP. Ces signaux peuvent être obtenus directement à l'aide des électrodes présentes dans la ou les sondes de détection sur le nerf vague (capture d'un signal composite ENG + EMG) ou encore entre le boitier de stimulation et l'une des électrodes périphériques sur la ou les sondes de détection.

De préférence, l'analyse électromyographique est réalisée en liaison avec l'étape 110 de la figure 2, et la stimulation est effectuée seulement dans le cas où une activité musculaire supérieure à un seuil est détectée en même temps que le phénomène de décharge massive ou antérieurement à ce phénomène.

En référence maintenant à la Figure 2, on va décrire un exemple préféré et simplifié d'un processus mis en oeuvre avec le système de la Figure 1.

A l'étape 100, l'unité 20, qui recueille périodiquement les signaux issus d'au moins l'une des sondes 10g et 10d, effectue un calcul de l'énergie globale des potentiels d'action dans le nerf vague respectif. Ce calcul est avantageusement effectué numériquement, les signaux faisant l'objet d'une conversion analogique/numérique dans le circuit 26. Il met en oeuvre avantageusement un filtrage passe-bande, par exemple sur une bande comprise entre environ 300 Hz et 3 kHz, un redressement et une intégration temporelle, de façon connue en soi dans le domaine du traitement de signal.

Aussi longtemps que cette énergie ne dépasse pas un seuil donné, le test est effectué à nouveau périodiquement par une commande en boucle fermé, par exemple avec une période de 0,5 à 10 secondes.

Lorsque l'unité 20 détecte que cette énergie est supérieure à un seuil, alors les autres paramètres physiologiques sont testés à l'étape 110, cette étape étant toutefois optionnelle. En particulier, l'unité 20 peut détecter une sortie de fenêtre de la fréquence cardiaque, un bloc auriculo-ventriculaire, une baisse de l'activité respiratoire en deçà d'un seuil ou encore une activité musculaire supérieure à un seuil comme expliqué plus haut.

Tant que ces paramètres restent dans les gammes normales, le processus est redirigé en amont de l'étape 100 et l'énergie globale des potentiels d'action est à nouveau surveillée.

Si l'étape 110, lorsqu'elle est prévue, confirme une anomalie cardiaque ou respiratoire, alors un cycle de dépolarisation et/ou d'hyperpolarisation des deux nerfs vagues à l'aide des sondes 10g, 10d est réalisé à l'étape 120 afin de neutraliser les potentiels d'action au moins dans le sens efférent dans les nerfs vagues, comme expliqué plus haut.

A la suite de ce cycle, qui peut consister à appliquer des salves de signaux de dépolarisation et/ou d'hyperpolarisation, alternatifs et asymétriques comme décrit plus haut, l'unité 20 exploite les signaux du capteur 31 pour déterminer à l'étape 130 si la fréquence cardiaque baisse en deçà d'un certain seuil, par exemple de l'ordre de 20 à 30 bpm selon le patient. Dans l'affirmative, cela signifie que le risque de décès est toujours élevé et un nouveau cycle de dépolarisation ou d'hyperpolarisation est mis en oeuvre, et ainsi de suite. Dans la négative, le risque de mort subite peut être écarté avec une certaine fiabilité et le processus est terminé et un nouveau processus de surveillance peut être engagé selon le cadencement défini dans l'unité 20.

On a représenté sur la Figure 3 à titre d'illustration le signal résultant du traitement par un filtrage passe-bande, puis une fonction quadratique (mise au carré), puis un filtrage passe-bas, d'un signal acquis à partir d'une sonde de détection sur un nerf vague. Ce signal résultant, intègre des éléments de l'électroneurogramme et d'électromyogramme, qui peuvent être toutefois séparés par filtrage et traitement supplémentaire du signal.

On observe sur la Figure 3 entre les instants ta et tb un phénomène de décharge massive. On comprend que par le traitement décrit ci-dessus, il est possible de détecter une décharge massive. C'est lorsque ce signal résultant dépasse un seuil donné qu'un risque de mort subite lors d'une crise d'épilepsie est identifié.

On a représenté sur la Figure 4 un exemple de signaux de stimulation appliqués à une sonde de nerf vague selon l'invention, dans le but de réaliser une hyperpolarisation du nerf dans le sens efférent.

Ces signaux comprennent une succession d'impulsions de courant positives P1 et d'impulsions de courant négatives P2 d'amplitudes et de durées différentes, l'impulsion P2 étant suivie d'une impulsion P2' de décharge passive, de façon connue en soi. Dans le cas d'espèce, les impulsions de courant positives ont une amplitude plus grande, mais une durée plus courte, que les impulsions négatives. Avantageusement, ces amplitudes et ces durées sont choisies de telle sorte que le bilan des charges électriques appliquées au nerf vague et soutirées du nerf vague soit neutre, afin d'éviter que la stimulation ne charge électriquement cette région corporelle.

De préférence, l'écart de durée entre les impulsions positives P1 et les impulsions négatives P2 est compris entre environ 1 et 4 µs, selon la fréquence du signal.

Cette fréquence est avantageusement comprise entre 15 et 300 kHz, et plus préférentiellement voisine de 20 kHz ou supérieure à 100 kHz afin de provoquer l'effet souhaité en minimisant l'énergie délivrée.

Ainsi, pour une fréquence de 20 kHz (soit une durée totale des impulsions de 50 µs (période TP sur la Figure 4), on peut prévoir par exemple une impulsion positive P1 d'une durée de 15 µs, une impulsion négative d'une durée de 18 µm, les 17 µm restantes étant occupées par l'impulsion de décharge passive et par un temps d'attente avant l'impulsion positive suivante.

Selon la nature et le type de la sonde, l'intensité électrique du signal de stimulation pourra varier, mais on utilise typiquement une amplitude d'intensité de l'ordre de 3 à 5 mA. Pour obtenir (au moins approximativement) la neutralité électrique susmentionnée, l'amplitude de chaque impulsion positive dans l'exemple numérique qui précède est 18/15 fois plus grande que l'amplitude de chaque impulsion négative.

Par ailleurs, la séquence d'impulsions P1, P2, P2' est répétée un certain nombre de fois sur une durée totale (TS sur la Figure 4) de l'ordre de quelques secondes, ou encore de façon continue jusqu'à la cessation de la décharge massive ou jusqu'à la reprise d'une fréquence cardiaque supérieure au seuil.

Dans l'exemple ci-dessus, la stimulation réalise une hyperpolarisation du nerf vague dans le sens efférent. En mettant en oeuvre des impulsions de courant négatives de plus grande amplitude que les impulsions de courant positives (avec de préférence encore des durées plus courtes), on peut en variante réaliser une dépolarisation des nerfs vagues dans le sens efférent.

Bien entendu, on pourra apporter de nombreuses variantes et modifications à l'invention. En particulier :
- la décharge massive susceptible d'indiquer une activité physiopathologique liée à une crise d'épilepsie susceptible d'engendrer un phénomène de mort subite peut être détectée dans un seul nerf vague ou dans les deux nerfs vagues, de façon identique ou différenciée ;
- l'action électrique visant à neutraliser les potentiels d'action peut être appliquée aux deux nerfs vagues de façon identique ou différenciée ;
- la ou les sondes utilisées pour la détection d'une décharge massive peuvent être les mêmes sondes que pour la neutralisation des potentiels d'action, ou des sondes différentes ;
- les critères et valeurs de paramètres de détection, ainsi que les paramètres d'action électrique de stimulation, peuvent être ajustés par essais cliniques afin d'optimiser l'efficacité de détection et d'action ;
- des paramètres physiologiques autres que la fréquence cardiaque, l'activité respiratoire et l'activité musculaire peuvent être utilisées pour, le cas échéant, conforter ou au contraire écarter le risque d'une mort subite induite par l'épilepsie.

## Revendications

1. Système destiné à contenir des risques de mort subite en cas de crise d'épilepsie, comprenant :
- une unité de détection et de commande (20) ;
- au moins une sonde de détection (10g, 10d) reliée à l'unité de détection et de commande et destinée à être appliquée sur au moins l'un des deux nerfs vagues d'un patient,
- des moyens (24) prévus dans l'unité de détection et de commande pour détecter un phénomène de décharge massive de potentiels d'action dans au moins un nerf vague en déterminant une densité d'énergie électrique globale dans au moins un nerf vague à partir des signaux délivrés par la ou les sondes de détection,
- au moins une sonde de stimulation d'un nerf vague, et
- des moyens (24) prévus dans l'unité de détection et de commande et aptes, en réponse à la détection d'une décharge massive, à appliquer à la ou aux sondes de stimulation (10g, 10d) des signaux de stimulation asymétriques prédéterminés aptes à provoquer une dépolarisation et/ou une hyperpolarisation du ou des nerfs vagues et à bloquer la conduction des potentiels d'action au moins dans le sens efférent.

2. Système selon la revendication 1, dans lequel la ou les sondes de détection et la ou les sondes de stimulation sont constituées par les mêmes sondes (10g, 10d).

3. Système selon la revendication 1 ou 2, dans lequel les moyens de détection de décharge massive (24) comprennent un moyen de comparaison d'une puissance électrique dans une bande de fréquences déterminée avec un seuil.

4. Système selon la revendication 3, dans lequel la puissance électrique est déterminée un filtrage passe-bande dans ladite bande de fréquences, suivi par une mise au carré et un filtrage passe-bas du signal en sortie du filtrage passe bande.

5. Système selon l'une des revendications 1 à 4, lequel comprend deux sondes de stimulation des nerfs vagues gauche et droit.

6. Système selon l'une des revendications 1 à 5, dans lequel les signaux de stimulation sont périodiques et déterministes.

7. Système selon la revendication 6, dans lequel les signaux de stimulation comprennent des impulsions positives plus courtes que les impulsions négatives, de manière à réaliser une dépolarisation des fibres nerveuses du ou des nerfs vagues.

8. Système selon la revendication 6, dans lequel les signaux de stimulation comprennent des impulsions positives plus longues que les impulsions négatives, de manière à réaliser une hyperpolarisation des fibres nerveuses du ou des nerfs vagues.

9. Système selon l'une des revendications 6 à 8, dans lequel les signaux périodiques asymétriques possèdent une fréquence comprise entre environ 15 et 300 kHz.

10. Système selon l'une des revendications 7 à 9, dans lequel les signaux de stimulation comprennent une succession d'impulsions de courant positives et négatives généralement rectangulaires, les amplitudes des impulsions positives et négatives étant différentes.

11. Système selon la revendication 10, dans lequel les impulsions de courant de plus grande amplitude sont plus courtes en durée que les impulsions de courant de plus petite amplitude.

12. Système selon la revendication 11, dans lequel les amplitudes et les durées sont choisies de manière à ce que le bilan des charges électriques appliquées aux électrodes et soutirées des électrodes soient essentiellement neutre.

13. Système selon l'une des revendications 10 à 12, dans lequel la durée (TS) des impulsions de stimulation est prédéterminée et comprise entre environ 0,5 et environ 2 s.

14. Système selon l'une des revendications 10 à 12, lequel comprend un moyen de détermination de la fréquence cardiaque, et dans lequel les impulsions sont appliquées jusqu'à la reprise d'une fréquence cardiaque supérieure à un seuil.

15. Système selon l'une des revendications 1 à 14, dans lequel l'unité de détection et de commande comporte également une entrée pour au moins un signal choisi parmi un signal cardiaque, un signal respiratoire, un signal d'oxymétrie et un signal d'électromyographie, le système comprenant dans ce dernier cas des moyens pour dériver le signal d'électromyographie du signal fourni par la ou les sondes de détection.

## Patentansprüche

1. System zur Eindämmung des Risikos eines plötzlichen Todes bei einem epileptischen Anfall, umfassend:
- eine Erfassungs- und Steuereinheit (20);
- mindestens einer Erfassungssonde (10g, 10d), die mit der Erfassungs- und Steuereinheit verbunden und dazu bestimmt ist, an mindestens einen der beiden Vagusnerven eines Patienten angelegt zu werden,
- Mittel (24), die in der Erfassungs- und Steuereinheit vorgesehen sind, um ein Phänomen der Massenentladung von Aktionspotentialen in mindestens einem Vagusnerv zu erfassen, indem aus den von der oder den Erfassungssonden gelieferten Signalen eine elektrische Gesamtenergiedichte in mindestens einem Vagusnerv ermittelt wird,
- mindestens eine Sonde zur Stimulation eines Vagusnervs, und
- Mittel (24), die in der Erfassungs- und Steuereinheit vorgesehen und geeignet sind, als Reaktion auf die Erfassung einer Massenentladung an die Stimulationssonde(n) (10g, 10d) vorbestimmte asymmetrische Stimulationssignale anzulegen, die geeignet sind, eine Depolarisation und/oder Hyperpolarisation des oder der Vagusnerven zu bewirken und die Weiterleitung von Aktionspotentialen zumindest in efferenter Richtung zu blockieren.

2. System nach Anspruch 1, bei dem die Erfassungssonde(n) und die Stimulationssonde(n) aus denselben Sonden (10g, 10d) bestehen.

3. System nach Anspruch 1 oder 2, wobei die Mittel zur Erkennung einer Massenentladung (24) ein Mittel zum Vergleich einer elektrischen Leistung in einem bestimmten Frequenzband mit einem Schwellenwert umfassen.

4. System nach Anspruch 3, wobei die elektrische Leistung durch eine Bandpassfilterung in dem Frequenzband bestimmt wird, gefolgt von einer Quadrierung und einer Tiefpassfilterung des Signals am Ausgang der Bandpassfilterung.

5. System nach einem der Ansprüche 1 bis 4, das zwei Sonden zur Stimulation des linken und rechten Vagusnervs umfasst.

6. System nach einem der Ansprüche 1 bis 5, wobei die Stimulationssignale periodisch und deterministisch sind.

7. System nach Anspruch 6, wobei die Stimulationssignale positive Impulse umfassen, die kürzer als die negativen Impulse sind, um eine Depolarisation der Nervenfasern des oder der Vagusnerven zu bewirken.

8. System nach Anspruch 6, wobei die Stimulationssignale positive Impulse umfassen, die länger als die negativen Impulse sind, um eine Hyperpolarisation der Nervenfasern des oder der Vagusnerven zu bewirken.

9. System nach einem der Ansprüche 6 bis 8, wobei die asymmetrischen periodischen Signale eine Frequenz zwischen etwa 15 und 300 kHz aufweisen.

10. System nach einem der Ansprüche 7 bis 9, wobei die Stimulationssignale eine Folge von allgemein rechteckigen positiven und negativen Stromimpulsen umfassen, wobei die Amplituden der positiven und negativen Impulse unterschiedlich sind.

11. System nach Anspruch 10, wobei die Stromimpulse größerer Amplitude in der Dauer kürzer sind als die Stromimpulse kleinerer Amplitude.

12. System nach Anspruch 11, wobei die Amplituden und die Dauer so ausgewählt sind, dass die Bilanz der elektrischen Ladungen, die an die Elektroden angelegt und von den Elektroden abgezogen werden, im Wesentlichen neutral ist.

13. System nach einem der Ansprüche 10 bis 12, wobei die Dauer (TS) der Stimulationsimpulse vorbestimmt ist und zwischen etwa 0,5 und etwa 2 s liegt.

14. System nach einem der Ansprüche 10 bis 12, das ein Mittel zur Bestimmung der Herzfrequenz umfasst, und bei dem die Impulse so lange angelegt werden, bis eine Herzfrequenz oberhalb eines Schwellenwerts wieder erreicht ist.

15. System nach einem der Ansprüche 1 bis 14, wobei die Erfassungs- und Steuereinheit auch einen Eingang für mindestens ein Signal aufweist, das aus einem Herzsignal, einem Atmungssignal, einem Oximetriesignal und einem Elektromyographiesignal ausgewählt ist, wobei das System im letzteren Fall Mittel zum Ableiten des Elektromyographie-Signals aus dem von der oder den Erfassungssonden gelieferten Signal aufweist.

## Claims

1. System intended to limit the risks of sudden death in the event of an epileptic seizure, comprising:
- a detection and control unit (20);
- at least one detection probe (10g, 10d) connected to the detection and control unit and intended to be applied to at least one of the two vagus nerves of a patient,
- means (24) provided in the detection and control unit for detecting a phenomenon of mass discharge of action potentials in at least one vagus nerve by determining a global electrical energy density in at least one vagus nerve from signals delivered by the detection probe(s),
- at least one stimulation probe for a vagus nerve, and
- means (24) provided in the detection and control unit that are capable, in response to the detection of a mass discharge, of applying predetermined asymmetric stimulation signals to the stimulation probe(s) (10g, 10d) capable of causing a depolarization and/or hyperpolarization of the vagus nerve(s) and of blocking the conduction of the action potentials at least in the efferent direction.

2. System according to claim 1, wherein the detection probe(s) and the stimulation probe(s) are made of the same probes (10g, 10d).

3. System according to one of claims 1 and 2, wherein the mass discharge detection means (24) are comprise a means for comparing an electrical power in a determined frequency band with a threshold.

4. System according to claim 3, wherein the electrical power is determined by a bandpass filtering in said frequency band, following by a squaring and a low-pass filtering of the signal at the output of the bandpass filtering.

5. System according to one of claims 1 to 4, which comprises two stimulation probes for the left and right vagus nerves.

6. System according to one of claims 1 to 5, wherein the stimulation signals are periodic and deterministic.

7. System according to claim 6, wherein the stimulation signals comprise positive pulses shorter than the negative pulses, so as to achieve a depolarization of the nerve fibers of the vagus nerve(s).

8. System according to claim 6, wherein the stimulation signals comprise positive pulses longer than the negative pulses, so as to achieve an hyperpolarization of the nerve fibers of the vagus nerve(s).

9. System according to one of claims 6 to 8, wherein the asymmetric periodic signals have a frequency of between around 15 and 300 kHz.

10. System according to one of claims 7 to 9, wherein the stimulation signals comprise a succession of generally rectangular positive and negative current pulses, the amplitudes of the positive and negative pulses being different.

11. System according to claim 10, wherein the current pulses of greater amplitude are shorter in duration than the current pulses of smaller amplitude.

12. System according to claim 11, wherein the amplitudes and durations are chosen so that the balance of the electrical charges applied to the electrodes and taken from the electrodes is essentially neutral.

13. System according to one of claims 10 to 12, wherein the duration (TS) of the stimulation pulses is predetermined and is comprised between around 0.5 and around 2 s.

14. System according to one of claims 10 to 12, comprising a means for heart rate determination, and wherein the pulses are applied until a heart rate above the threshold is resumed.

15. System according to one of claims 1 to 14, wherein the detection and control unit further comprises an input for at least one signal chosen from a cardiac signal, a respiratory signal, an oximetry signal and an electromyography signal, the system comprising in the latter case means for deriving the electromyography signal from the signal provided by the detection probe(s).
